# FASCICULE DE BREVET EUROPEEN

(11) **EP 2 016 955 B1**
(45) Date de publication et mention de la délivrance du brevet: **25.01.2012**
(21) Numéro de dépôt: 08356076.3
(22) Date de dépôt: 02.06.2008
(51) Int. Cl.: A61L 2/07, A61L 2/22

(54) **Dispositif et procédé de désinfection de surfaces**
Oberflächendesinfektionsvorrichtung und -verfahren
Device and method for disinfecting surfaces

(30) Priorité: 16.07.2007 FR 0705130
(43) Date de publication de la demande: 21.01.2009
(73) Titulaire: Sanivap, 69760 Limonest (FR)
(72) Inventeur: Piot, Jacques, 69380 Chazay d'Azergues (FR)
(74) Mandataire: Brédeville, Odile Marie

(56) Documents cités:
- EP-A- 0 437 155
- EP-A- 1 586 262
- WO-A-01/37886
- WO-A-2007/021416
- FR-A- 2 721 539
- JP-A- 4 189 727

## Description

La présente invention concerne un dispositif transportable de désinfection de surfaces et un procédé pour désinfecter des surfaces à l'aide d'un tel dispositif.

De nombreux professionnels des secteurs médicaux, alimentaires, agroalimentaires, des secteurs du transport, de l'hôtellerie et de la restauration, des industries diverses comme l'industrie pharmaceutique, sont confrontés aujourd'hui à de multiples problèmes d'hygiène et de désinfection. Ainsi, il est connu d'utiliser de la vapeur pour nettoyer des endroits publics tels que les hôpitaux, les lieux de restauration collective, les cuisines collectives, les hôtels, les transports en commun, etc...

L'utilisation de la vapeur d'eau seule pour la désinfection apporte de nombreux avantages en terme d'efficacité et d'absence d'utilisation de produits chimiques polluants. Toutefois, les procédés connus ne donnent pas toujours entière satisfaction. Des protocoles de nettoyage ou de désinfection précis, par exemple avec un temps minimum de contact de la vapeur avec la surface à nettoyer ou encore une distance maximum entre le jet de vapeur d'eau et la surface à traiter, doivent être respectés.

Par ailleurs, les procédés connus, par ex. EP 1 586 262, présentent des limites d'efficacité biocide vis-à-vis de certaines bactéries particulièrement résistantes, comme par exemple les formes sporulées de bactéries. Comme bactéries particulièrement résistantes, pour lesquelles les procédés de désinfection classiques ne sont pas assez efficaces, on peut citer notamment les spores de Clostridium difficile.

Il existe des dispositifs de désinfection d'articles destinés à l'industrie pharmaceutique ou médicale, par ex. FR 2 721 539 : ces dispositifs de désinfection sont généralement sous la forme d'une ou plusieurs enceintes au sein desquelles on dispose les articles à désinfecter : de la vapeur et un désinfectant sont alors injectés au sein de ces enceintes pour procéder à la désinfection des articles. Dans de tels dispositifs, afin de réaliser la meilleure désinfection possible, on utilise généralement un taux de désinfectant très élevé, qui serait toxique pour l'homme si le traitement était effectué à l'air libre. Les enceintes sont donc généralement fermées et étanches et la désinfection se fait en milieu fermé.

Toutefois, lorsque les surfaces à désinfecter sont par exemple des murs, des sols ou encore des plafonds, il n'est pas possible de réaliser le traitement de désinfection en milieu fermé. Par ailleurs, l'importance des surfaces à traiter exclut d'utiliser un dispositif immobile. Il n'est pas possible non plus de déplacer la surface à traiter par rapport au dispositif de désinfection.

La présente invention vise à remédier à ces inconvénients.

La présente invention porte sur un dispositif transportable de désinfection d'une surface, comprenant :
- au moins un réservoir d'eau,
- au moins un générateur de vapeur d'eau destiné à produire un flux de vapeur d'eau,
- au moins un réservoir d'une composition désinfectante comprenant au moins un désinfectant,
- au moins un injecteur de ladite composition désinfectante dans ledit flux de vapeur d'eau pour réaliser un mélange sous la forme d'un jet de vapeur d'eau et de composition désinfectante, ledit injecteur comprenant en outre une chambre de préchauffage et de micro pulvérisation de ladite composition désinfectante, ledit injecteur étant intégré au générateur de vapeur d'eau, caractérisé en ce qu'il comprend en outre au moins un conduit flexible relié au générateur d'eau et apte à délivrer ledit mélange sous forme de jet de vapeur et de ladite composition désinfectante à une distance prédéterminée de ladite surface.

La présente invention augmente le pouvoir désinfectant de la vapeur d'eau tout en simplifiant les protocoles de désinfection en terme de facilité d'application et de rapidité. Elle n'utilise aucun produit chimique ayant un impact défavorable sur l'environnement et les utilisateurs.

Le dispositif selon l'invention permet de délivrer un mélange sous la forme d'un jet de vapeur et de composition désinfectante, un tel jet ayant un pouvoir désinfectant particulièrement efficace. En particulier, avec le dispositif selon l'invention, la diffusion de la composition désinfectante est assurée par le biais du jet de vapeur d'eau. La vapeur d'eau potentialise, par sa température et sa capacité de dispersion, l'effet désinfectant de la composition désinfectante. Elle permet en particulier d'obtenir une efficacité de désinfection démontrée à partir de dosage réduit de désinfectant dans la composition désinfectante.

Le dispositif selon l'invention est transportable : par le terme « transportable », on entend, au sens de la présente invention que le dispositif selon l'invention est mobile et peut facilement être transporté ou déplacé d'un endroit à un autre par un seul être humain. Un tel dispositif transportable peut être déplacé sur l'ensemble de la surface d'un mur ou d'un sol, quelle que soit cette surface.

Par exemple, la surface à désinfecter avec le dispositif selon l'invention et/ou selon le procédé de l'invention, peut présenter une aire significative, notamment supérieure ou égale à 1 ou 10 m², et le dispositif selon l'invention, du fait qu'il est transportable, peut être facilement déplacé par l'utilisateur afin que le conduit flexible délivre le mélange sous la forme d'un jet de vapeur d'eau et de composition désinfectante sur l'ensemble de ladite surface.

En particulier, grâce au dispositif selon l'invention, il est possible de diriger le jet de vapeur et de composition désinfectante et d'ajuster ce jet vis-à-vis de la surface à traiter à la distance désirée par l'utilisateur. L'utilisateur contrôle manuellement la direction du jet et la distance séparant ce jet de la surface à traiter.

La dispersion assurée par le jet de vapeur est parfaite : il est ainsi possible de traiter très simplement et rapidement toute surface ou équipement, même sans contact direct avec ladite surface ou ledit équipement à traiter et avec une durée de projection du mélange de jet de vapeur et de composition désinfectante très court. Les surfaces ou équipements à traiter, c'est-à-dire à désinfecter peuvent par exemple être des murs, des plafonds, des sols, des appareils divers (équipements médicaux tels que lits, incubateurs de néonatologie, tables d'opération, équipements alimentaires tels que chaînes de conditionnement, matériels de découpe, équipements industriels tels que chaînes de conditionnement et machines diverses utilisées en industrie, par exemple en pharmacie, en cosmétique...).

Par « désinfectant », on entend au sens de la présente demande un composé possédant un pouvoir biocide, selon les normes applicables au procédé de l'invention (ie tests sur supports) NF EN 14561-14562-14563, ASTM-E-1053-97 ou inspirées de la norme EN-14347.

Par « désinfection », on entend au sens de la présente demande un traitement permettant d'obtenir des résultats au moins conformes aux normes applicables au procédé de l'invention (ie tests sur supports) NF EN 14561-14562-14563, ASTM-E-1053-97 ou inspirées de la norme EN-14347.

Par « distance prédéterminée », on entend au sens de la présente demande, une distance voulue par l'utilisateur, pour réaliser la meilleure désinfection possible. A titre d'exemple, cette distance pourra varier de 1 à 100 cm.

Le conduit flexible peut être muni d'une poignée de commande. Il est possible de fixer sur cette poignée de commande différents accessoires pour délivrer ledit mélange de jet de vapeur d'eau et de ladite composition désinfectante. Grâce au dispositif selon l'invention, il n'est pas nécessaire que l'extrémité du conduit flexible délivrant le mélange sous la forme d'un jet de vapeur d'eau et de composition désinfectante soit au contact de la surface à traiter. L'extrémité du conduit flexible peut être à distance, par exemple à 50 cm de la surface à traiter tout en assurant une désinfection parfaite.

Ainsi, dans une forme de réalisation, ledit conduit flexible est muni d'une poignée de commande commandant l'ouverture et/ou la fermeture, d'une part du jet de vapeur d'eau, d'autre part, de l'injection de composition désinfectante dans le jet de vapeur d'eau.

De préférence, ladite composition désinfectante est sous la forme liquide avant injection dans ledit flux de vapeur d'eau.

Dans une forme de réalisation de l'invention, ledit désinfectant est choisi parmi le peroxyde d'hydrogène, un mélange d'acide acétique et d'acide peracétique, l'acide lactique, les huiles essentielles, et leurs mélanges.

De préférence, ledit désinfectant est le peroxyde d'hydrogène. L'emploi du peroxyde d'hydrogène assure une biodégradabilité de la composition, après utilisation, totale et rapide. Une telle composition permet de préserver l'environnement.

Dans une forme de réalisation de l'invention, le désinfectant est présent dans la composition désinfectante à une teneur allant de 3 à 15%, en poids, de préférence de 3 à 10%, en poids, de préférence encore de 5 à 10%, en poids, de préférence encore de 8 à 10%, en poids, par rapport au poids de la composition. De préférence, ladite composition désinfectante est une solution aqueuse dudit désinfectant.

Ainsi, dans une forme de réalisation de l'invention, la composition désinfectante est une solution aqueuse de peroxyde d'hydrogène. Une telle solution présente de plus l'avantage d'être rapidement biodégradable entièrement sans laisser aucune trace résiduelle.

La présente invention porte encore sur un procédé de désinfection d'au moins une surface comprenant au moins les étapes suivantes :
- a°) on dispose d'un dispositif tel que décrit ci-dessus,
- b°) on génère un flux de vapeur d'eau,
- c°) on préchauffe et on micro pulvérise ladite composition désinfectante,
- d°) on injecte dans le flux de vapeur d'eau ladite composition désinfectante comprenant au moins un désinfectant, sous forme préchauffée et micro pulvérisée, pour obtenir un mélange sous la forme d'un jet de vapeur d'eau et de composition désinfectante,
- e°) on projette ledit mélange sous forme de jet de vapeur d'eau et de la composition désinfectante à une distance prédéterminée de ladite surface à l'aide dudit conduit flexible.

Le procédé selon l'invention est non toxique, non corrosif. En particulier, il s'utilise à l'air libre, en présence humaine, sans nécessiter de protection particulière contraignante pour l'utilisateur autre que éventuellement des accessoires classiques de protection tels que masque, lunettes et gants en fonction de la composition désinfectante utilisée et de son dosage en désinfectant.

Dans une forme de réalisation de l'invention, le générateur de vapeur d'eau dudit dispositif comprenant une chaudière et une électrovanne destinée à régler le débit du, et plus particulièrement à commander le, flux de vapeur d'eau sortant de ladite chaudière, à l'étape b°), la pression de la vapeur d'eau est portée à au moins 2,5 10⁵ Pa (2,5 bars) dans ladite chaudière en utilisation continue.

Dans une forme de réalisation de l'invention, à l"étape b°), le flux de vapeur d'eau est porté à un débit d'au moins 30 g/minute, par exemple d'au moins 40 g/minute, ou encore d'au moins 50 g/minute. Par exemple, le flux de vapeur d'eau présente une température d'au moins 130°C dans ladite chaudière en utilisation continue.

Dans une forme de réalisation de l'invention, à l'étape c°), la composition désinfectante est préchauffée à une température supérieure ou égale à 70°C.

Dans une forme de réalisation de l'invention, lors de l'étape d°), le flux de la composition désinfectante est porté à un débit d'au moins 30 g/minute, par exemple d'au moins 60 g/minute.

De préférence, le débit du mélange sous la forme du jet de vapeur d'eau et de composition désinfectante est d'au moins 60 g/minute, de préférence d'au moins 80 g/minute, par exemple d'au moins 90 g/minute. De préférence, la température du mélange sous la forme du jet de vapeur d'eau et de composition désinfectante est d'au moins 100°C, de préférence d'au moins 110°C, par exemple d'au moins 120°C au niveau de l'injecteur dudit dispositif.

Dans une forme de réalisation de l'invention, selon l'étape e°), on projette ledit mélange pendant une durée allant de 1 à 5 secondes.

Grâce aux dispositif et procédé selon l'invention, la désinfection d'une surface peut être réalisée en un temps très court.

Dans une forme de réalisation, selon l'étape e°), le mélange sous forme de jet est projeté sur la surface à traiter à une distance allant de 10 cm à 100 cm, par exemple allant de 10 cm à 50 cm. Ainsi, il est extrêmement facile de désinfecter efficacement une surface au moyen du dispositif et du procédé selon l'invention.

Selon le dispositif et le procédé de l'invention, La composition désinfectante se vaporise dans le jet vapeur au niveau de l'injecteur. Ceci assure une excellente homogénéité du mélange vapeur d'eau - composition désinfectante et un transport ultérieur du mélange, par exemple dans le conduit flexible, très rapide assurant de préférence un maintien sous forme de vapeur jusqu'à la délivrance du jet, par exemple au niveau d'une poignée ou autres accessoires.

De préférence, le débit et la température de la vapeur d'eau ainsi que le débit, le dosage et la température de préchauffage de la composition désinfectante fixés par le procédé garantissent la composition et la nature du mélange obtenu.

Le mélange de jet de vapeur et de composition désinfectante obtenu grâce au dispositif selon l'invention et selon le procédé de l'invention a un excellent pouvoir nettoyant. Il dissout les graisses, décolle les résidus, impuretés, souillures et protéines présents sur la surface, agit comme un tensioactif et nettoie en profondeur. Simultanément, il possède un pouvoir désinfectant, en particulier selon les normes applicables au procédé de l'invention (ie tests sur supports) NF EN 14561-14562-14563, ASTM-E-1053-97 ou inspirées de la norme EN-14347. Le mélange de jet de vapeur et de composition désinfectante obtenu grâce au dispositif selon l'invention et selon le procédé de l'invention a ainsi une action biocide, bactéricide, fongicide, mycobactéricide, virucide et sporicide.

Dans une forme de réalisation du procédé de l'invention, la composition désinfectante est une solution aqueuse de peroxyde d'hydrogène, ledit peroxyde d'hydrogène étant présent dans la solution aqueuse à une teneur de 10%, en poids, par rapport au poids de ladite solution aqueuse, à l'étape b°) le flux de vapeur d'eau est porté à un débit de 40 g/ minute, et à l'étape d°) le flux de la composition désinfectante est porté à un débit de 60 g/minute.

Par ailleurs, le procédé selon l'invention, en particulier compte tenu de la concentration en désinfectant dans la composition désinfectante et des débits respectifs préférés pour le flux de vapeur et pour la composition désinfectante, permet d'effectuer une désinfection optimale de toute surface, en particulier d'une surface présentant une aire significative, à l'air libre, en présence humaine, sans présenter de danger pour l'utilisateur, et ce sans que ce dernier ait à prendre des mesures de protection particulière, autres qu'éventuellement masque, gants et lunettes en fonction du désinfectant utilisé et de son dosage dans la composition désinfectante.

Les avantages de la présente invention apparaîtront plus clairement au vu de la description ci-après et des dessins annexés dans lesquels :
- la Figure 1 est un schéma décrivant les différentes étapes d'un exemple de réalisation d'un procédé selon l'invention,
- la Figure 2 est une vue en perspective d'un exemple de réalisation d'un dispositif selon l'invention.

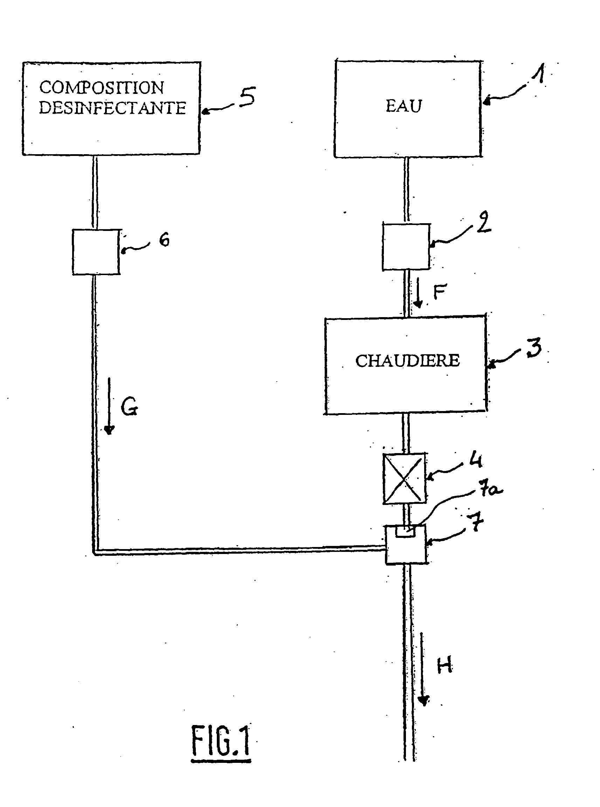

En référence à la figure 1 sont décrites les différentes étapes d'un procédé selon l'invention. Selon le procédé de la figure 1, on dispose d'un réservoir d'eau 1. Ce réservoir d'eau 1 est directement relié à une pompe 2, elle-même reliée à une chaudière 3. L'eau du réservoir d'eau 1 est pompée dans la chaudière 3 à l'aide de la pompe 2, selon la flèche F représentée sur la figure 1. L'eau est transformée en flux de vapeur d'eau par chauffage dans la chaudière 3.

Selon le procédé décrit à la figure 1, on dispose également d'une électrovanne 4 située en aval de la chaudière 3 et commandée à partir d'une poignée de commande (non représentée sur la figure 1), permettant de commander la sortie du flux de vapeur d'eau de la chaudière 3.

Selon le procédé décrit à la figure 1, on dispose d'un réservoir 5 d'une composition comprenant au moins un désinfectant. La composition désinfectante comprend de préférence un désinfectant choisi parmi le peroxyde d'hydrogène, un mélange d'acide acétique et d'acide peracétique, l'acide lactique, les huiles essentielles, et leurs mélanges. De préférence, le désinfectant est le peroxyde d'hydrogène.

La composition désinfectante est de préférence sous la forme d'une solution liquide du désinfectant. De préférence, la composition désinfectante est une solution aqueuse de peroxyde d'hydrogène. Une telle solution aqueuse présente l'avantage d'être très rapidement totalement biodégradable en ne laissant aucune trace résiduelle. Le procédé selon l'invention est ainsi non toxique pour l'environnement.

Comme montré sur la figure 1, la composition désinfectante du réservoir 5 est pompée selon la flèche G à l'aide d'une pompe 6 dans un injecteur 7 situé en aval de l'électrovanne 4 et réalisant l'injection de la composition désinfectante dans le flux de vapeur sortant de l'électrovanne 4. Cet injecteur 7 comporte une chambre 7a assurant le pré-chauffage et la micro pulvérisation de la composition désinfectante dans le flux vapeur. Par exemple, la composition désinfectante est préchauffée à une température supérieure ou égale à 70 °C. La pompe 6 est commandée à partir de la poignée de commande.

Le mélange sortant de l'injecteur 7, représenté par la flèche H sur la figure 1, est sous la forme d'un jet de vapeur d'eau et de composition désinfectante. De préférence, ce jet présente un débit d'au moins 60 g/minute, ou encore d'au moins 80 g/minute ou d'au moins 90 g/minute et une pression d'au moins 2,5 10⁵ Pa (2,5 bars) dans la chaudière 3 en utilisation continue.

Pour mettre en oeuvre le procédé selon l'invention, on projette ce jet sur la surface à traiter. Ce jet peut être projeté pendant un temps allant de 1 à 5 secondes, et à une distance inférieure à 50 cm mais sans contact direct nécessaire avec la surface.

La diffusion de la composition désinfectante est parfaite. Les surfaces à traiter sont désinfectées de façon simple et très rapide.

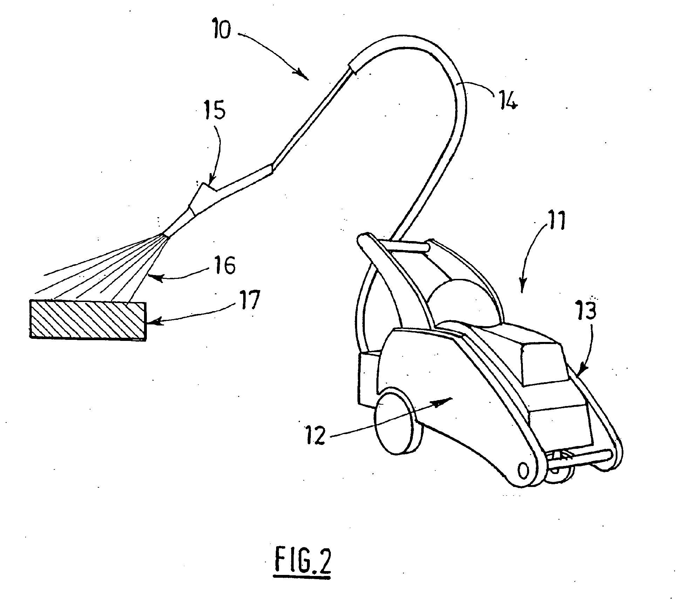

Sur la figure 2 est représenté un exemple de dispositif de désinfection 10 selon l'invention. Le dispositif 10 représenté sur la figure 2 comprend un générateur de vapeur d'eau 11 comprenant un réservoir d'eau 12 intégré. Ce générateur comprend une chaudière et une électrovanne et produit un flux de vapeur d'eau, par exemple sous une pression de 2,5 10⁵ Pa (2,5 bars) minimum dans la chaudière en utilisation continue.

Le générateur de vapeur d'eau 11 de la figure 2 comprend également un réservoir 13 pour la composition désinfectante. Le réservoir 13 de composition désinfectante est également intégré au générateur de vapeur d'eau 11. Un injecteur destiné à injecter la composition désinfectante, de préférence sous forme liquide, dans le flux de vapeur d'eau généré par le générateur de vapeur d'eau, est également intégré au générateur de vapeur d'eau 11. A la sortie de l'injecteur, on obtient un jet de vapeur d'eau et de composition désinfectante, par exemple sous une pression de 2,5 10⁵ Pa (2,5 bars) à une température supérieure à 100°C, de préférence supérieure à 110°C, ou encore supérieure à 120°C.

Le dispositif 10 de désinfection de la figure 2 comprend un conduit flexible 14 relié au générateur de vapeur d'eau 11, au travers duquel circule le mélange sous forme de jet de vapeur d'eau et de composition désinfectante. Le conduit flexible 14 est muni d'une poignée de commande 15 elle-même munie de deux interrupteurs servant à ouvrir ou à fermer :
- d'une part le jet de vapeur d'eau,
- d'autre part, l'injection de composition désinfectante dans le jet de vapeur d'eau.

Sur la poignée de commande, il peut être fixé une série d'accessoires adapté à la surface à traiter (brosse, diffuseur, balai vapeur, raclette, lance... ).

Ainsi, pour traiter une surface 17, un opérateur appuie sur les interrupteurs de la poignée de commande 15 pour projeter le mélange sous forme de jet 16 de vapeur d'eau et de composition désinfectante sur ladite surface 17. Lors de cette opération, le contact mécanique de l'extrémité du flexible 14 par laquelle sort le jet 16 avec la surface 17 à traiter n'est pas nécessaire. De préférence, la distance de projection est d'au plus 100 cm, par exemple de 50 cm. Le jet 16 présente un pouvoir désinfectant particulièrement efficace, par exemple conforme aux exigences des normes applicables au procédé de l'invention (ie tests sur supports) NF EN 14561-14562-14563, ASTM-E-1053-97 ou inspirées de la norme EN-14347.

La surface 17 est ainsi parfaitement traitée et ce en moins de 5 secondes par mètre carré.

En particulier, avec le dispositif selon l'invention, la diffusion de la composition désinfectante est assurée par le biais du jet de vapeur et cette diffusion est parfaite : il est ainsi possible de traiter très simplement et rapidement toute surface.

### EXEMPLES

### EXEMPLE 1 :

On réalise le traitement selon le procédé de l'invention d'une surface en inox de 1m² contaminée avec une population bactérienne de 7 Logs de Enterococcus hirae en présence de matières interférentes

Pour cela, on utilise un dispositif selon l'invention dans lequel le flux de vapeur d'eau a un débit de 50 g/minute et une température minimum de 130°C en utilisation continue au niveau de la chaudière.

On injecte dans ce flux de vapeur d'eau une composition désinfectante de péroxyde d'hydrogène stabilisé dosée à 4% en poids, par rapport au poids de la composition.

On obtient un mélange de jet vapeur d'eau - composition désinfectante sous forme vapeur à 120°C au niveau de l'injection et présentant un débit de 90 g/minute.

On traite la surface par projection du jet ci-dessus à une distance de 50 cm de la surface pendant 5 secondes.

Une fois le jet projeté, après un temps de contact de 15 min de la composition désinfectante avec la surface traitée, on obtient une réduction de la population microbienne de 6 Logs.

### EXEMPLE 2 :

On a effectué la désinfection de surfaces expérimentalement contaminées par des spores de *Clostridium difficile* (méthode des porte-germes) selon le procédé de l'invention, après deux passages (durée d'application du jet de vapeur et de composition désinfectante d'environ 1 seconde) et un temps de contact de 15 minutes.

Trois types de souches de *C*. *difficile* sont étudiés :
- la souche de PCR ribotype 027 (souche 1067) (souche hypervirulente épidémique)(S1)
- la souche VPI 10463 (toxinotype 0, souche de référence ) (S2)
- la souche CD196 (souche ATCC 43 596, PCR ribotype 027 non épidémique) (S3)

Deux types de matériaux de surface sont traités : M1 (résine vinylique) et M2 (plaque de stratifié). Ces matériaux sont nettoyés et stérilisés avant usage.

Les matériaux des surfaces à traiter (M1 et M2) sont expérimentalement contaminés par 0,1 ml d'une suspension de spores de *C*. *difficile* (environ 10⁶ spores/ml) préparée selon la technique de Wullt *et al*. (Wullt M, Odenholt I, Walder M. Activity of three disinfectants and acidified nitrite against Clostridium difficile spores. Infect Control Hosp Epidemiol 2003;24(10):765-8). Les matériaux sont déposés sur les supports porte-germes. Ces supports sont soumis au procédé de l'invention dans lequel :
- le flux de vapeur est de : 40 grammes/minute
- le flux de composition désinfectante est de : 60 grammes/minute
- la composition de la composition désinfectante est de : 10% H₂O₂ - 90% eau pure en poids

Après avoir neutralisé les résidus de désinfectants (0,02% de catalase [Sigma]), la quantité de bactéries présentes sur chaque matériau est numérée (N1) et comparée à la quantité de bactéries présentes sur le même type de matériau non exposé au produit désinfectant (N0).

Préparation de « témoins » :
La numération des spores présentes sur les différents types de matériau « témoins » est réalisée en immergeant et en sonicant les pièces à analyser dans 3 ml d'eau physiologique + neutralisant. Chaque suspension (0.1 ml de suspension pure et diluée jusqu'à 10 ⁻⁴) est ensuite ensemencée sur milieu TCCA (taurocholate cyclosérine, cefoxitine agar) et incubée 48 heures en anaérobiose.

Pour les matériaux testés (M1 et M2), 1 ml de la suspension est ensemencé sur 2 géloses TCCA afin d'avoir un seuil de sensibilité de la méthode de 3 spores /pièce.

Pour chaque souche et chaque matériau, les essais seront répétés 3 fois.

La quantité de spores est exprimée en log₁₀ d'UFC/ml.

Pour chaque essai, le facteur de réduction (FR) est calculé en faisant la différence entre le nombre de spores sur la pièce témoin et le nombre de spores sur la pièce soumise au procédé de désinfection selon l'invention.

Pour le Clostridium difficile forme sporulée, on obtient un facteur de réduction supérieur à 4.0 Log10.

Un tel résultat est conforme aux exigences des normes applicables au procédé de l'invention (ie tests sur supports) NF EN 14561-14562-14563, ASTM-E-1053-97 ou inspirées de la norme EN-14347.

### EXEMPLE 3 :

On a répété le procédé de l'exemple 2 mais avec les conditions suivantes :
- Flux de vapeur : 50 grammes/minute
- Flux de composition désinfectante : 50 grammes/minute
- Composition de la composition désinfectante : 6% H²O² - 94% eau pure en poids

Pour la Clostridium difficile forme sporulée, on obtient un facteur de réduction inférieur à 3.0 Log10.

### EXEMPLE 4 :

On a répété le procédé de l'exemple 2 sur les souches suivantes :
- Pseudomonas aeruginosa
- Echerichia coli
- Staphilococcus aureus

### Les résultats obtenus sont les suivants :

- Pseudomonas aeruginosa : facteur de réduction supérieur à 5.6 Log 10.
- Echerichia coli : facteur de réduction supérieur à 5.4 Log10.
- Staphilococcus aureus : facteur de réduction supérieur à 5.9 Log10.

Ces résultats sont conformes aux exigences des normes applicables au procédé de l'invention (ie tests sur supports) NF EN 14561-14562-14563-ASTM-E-1053-97 ou inspirées de la norme EN-14347.

### EXEMPLE 5 :

On a répété le procédé de l'exemple 3 sur les souches suivantes :
- Pseudomonas aeruginosa
- Echerichia coli
- Staphilococcus aureus

### Les résultats obtenus sont les suivants :

- Pseudomonas aeruginosa : facteur de réduction inférieur à 3.0 Log10.
- Echerichia coli : facteur de réduction inférieur à 3.0 Log10.
- Staphilococcus aureus : facteur de réduction inférieur à 3.0 Log 10.

## Revendications

1. Dispositif (10) transportable de désinfection d'une surface, comprenant :
- au moins un réservoir d'eau (1 ; 12),
- au moins un générateur (3; 11) de vapeur d'eau destiné à produire un flux de vapeur d'eau,
- au moins un réservoir (5 ; 13) d'une composition désinfectante comprenant au moins un désinfectant,
- au moins un injecteur (7) de ladite composition désinfectante dans ledit flux de vapeur d'eau pour réaliser un mélange sous la forme d'un jet (16) de vapeur d'eau et de composition désinfectante, ledit injecteur comprenant en outre une chambre de préchauffage et de micro pulvérisation de ladite composition désinfectante, ledit injecteur étant intégré au générateur de vapeur d'eau,
**caractérisé en ce qu'**il comprend en outre au moins un conduit flexible relié au générateur d'eau et apte à délivrer ledit mélange sous forme de jet de vapeur et de ladite composition désinfectante à une distance prédéterminée de ladite surface.

2. Dispositif (10) selon la revendication 1, **caractérisé en ce que** ledit conduit flexible (14) est muni d'une poignée de commande (15) commandant l'ouverture et/ou la fermeture, d'une part du jet de vapeur d'eau, d'autre part, de l'injection de composition désinfectante dans le jet de vapeur d'eau.

3. Dispositif (10) selon l'une quelconque des revendications 1 ou 2, **caractérisé en ce que** ledit désinfectant est choisi parmi le peroxyde d'hydrogène, un mélange d'acide acétique et d'acide peracétique, l'acide lactique, les huiles essentielles, et leurs mélanges, et est de préférence le peroxyde d'hydrogène.

4. Dispositif (10) selon l'une quelconque des revendications précédentes, **caractérisé en ce que** le désinfectant est présent dans la composition désinfectante à une teneur allant de 3 à 15% en poids, de préférence de 3 à 10%, en poids, de préférence encore de 5 à 10%, en poids, de préférence encore de 8 à 10% en poids, par rapport au poids de la composition.

5. Dispositif (10) selon l'une quelconque des revendications précédentes, **caractérisé en ce que** ladite composition désinfectante est une solution aqueuse de peroxyde d'hydrogène.

6. Procédé de désinfection d'au moins une surface comprenant au moins les étapes suivantes :
- a°) on dispose d'un dispositif (10) selon l'une quelconque des revendications 1 à 5,
- b°) on génère un flux de vapeur d'eau,
- c°) on préchauffe et on micro pulvérise ladite composition désinfectante,
- d°) on injecte dans le flux de vapeur d'eau ladite composition désinfectante comprenant au moins un désinfectant, sous forme préchauffée et micro pulvérisée, pour obtenir un mélange sous la forme d'un jet (16) de vapeur d'eau et de composition désinfectante,
- e°) on projette ledit mélange sous forme de jet de vapeur d'eau et de la composition désinfectante à une distance prédéterminée de ladite surface à l'aide dudit conduit flexible.

7. Procédé selon la revendication précédente, **caractérisé en ce que**, le générateur de vapeur d'eau dudit dispositif (10) comprenant une chaudière (3) et une électrovanne (4) destinée à commander le flux de vapeur d'eau sortant de ladite chaudière (3), à l'étape b°), la pression de la vapeur d'eau est portée à au moins 2,5 10⁵ Pa dans ladite chaudière (3) en utilisation continue

8. Procédé selon la revendication 6 ou 7, **caractérisé en ce que** à l"étape b°), le flux de vapeur d'eau est porté à un débit d'au moins 30 g/minute, par exemple d'au moins 40 g/minute, ou encore d'au moins 50 g/minute.

9. Procédé selon l'une des revendications 7 ou 8, **caractérisé en ce que** le flux de vapeur d'eau présente une température d'au moins 130°C dans ladite chaudière (3) en utilisation continue.

10. Procédé selon l'une quelconque des revendications 6 à 9, **caractérisé en ce que** le débit du mélange sous la forme du jet (16) de vapeur d'eau et de composition désinfectante est d'au moins 60 g/minute, de préférence d'au moins 80 g/minute, par exemple d'au moins 90 g/minute.

11. Procédé selon l'une quelconque des revendications 6 à 9, **caractérisé en ce que** la température du mélange sous la forme du jet (16) de vapeur d'eau et de composition désinfectante est d'au moins 100°C, de préférence d'au moins 110°C, par exemple d'au moins 120°C, au niveau de l'injecteur dudit dispositif (10).

12. Procédé selon l'une quelconque des revendications 6 à 11, **caractérisé en ce que**, selon l'étape d°), on projette ledit mélange sous forme de jet (16) pendant une durée allant de 1 à 5 secondes.

13. Procédé selon l'une quelconque des revendications 6 à 12, **caractérisé en ce que**, selon l'étape d°), le mélange sous forme de jet (16) est projeté sur la surface à traiter à une distance allant de 10 cm à 100 cm, par exemple allant de 10 cm à 50 cm.

14. Procédé selon l'une quelconque des revendications 6 à 13, **caractérisé en ce que**, lors de l'étape d°), le flux de la composition désinfectante est porté à un débit d'au moins 30 g/minute, par exemple d'au moins 60 g/minute.

15. Procédé selon l'une quelconque des revendications 6 à 14, **caractérisé en ce que** la composition désinfectante est une solution aqueuse de peroxyde d'hydrogène, ledit peroxyde d'hydrogène étant présent dans la solution aqueuse à une teneur de 10%, en poids, par rapport au poids de ladite solution aqueuse, à l'étape b°) le flux de vapeur d'eau est porté à un débit de 40 g/ minute, et à l'étape d°) le flux de la composition désinfectante est porté à un débit de 60 g/minute.

## Claims

1. A portable device (10) for disinfecting surfaces, comprising:
- at least one water tank (1; 12),
- at least one water vapor generator (3; 11) for producing a water vapor flow,
- at least one tank (5; 13) of a disinfecting composition comprising at least one disinfectant,
- at least one injector (7) of said disinfecting composition into said water vapor flow for making a mixture in the form of a jet (16) of water vapor and disinfecting composition, said injector further comprising a chamber for preheating and microatomizing said disinfecting composition, said injector being integrated into the water vapor generator,
**characterized in that** it further comprises at least one flexible tube connected to the water generator and capable of delivering said mixture in the form of a jet of water and said disinfecting composition at a predetermined distance from said surface.

2. The device (10) according to claim 1, **characterized in that** said flexible tube (14) is provided with a control handle (15) controlling the opening and/or closing, on the one hand of the water vapor jet, on the other hand of the injection of the disinfecting composition into the water vapor jet.

3. The device (10) according to any of claims 1 or 2, **characterized in that** said disinfectant is chosen from hydrogen peroxide, a mixture of acetic acid and peracetic acid, lactic acid, essential oils, and mixtures thereof, and preferably is hydrogen peroxide.

4. The device (10) according to any of the preceding claims, **characterized in that** the disinfectant is present in the disinfecting composition in a content ranging from 3 to 15 % in weight, preferably from 3 to 10 % in weight, more preferably from 5 to 10 % in weight, more preferably from 8 to 10 % in weight, with respect to the weight of the composition.

5. The device (10) according to any of the preceding claims, **characterized in that** said disinfecting composition is an aqueous solution of hydrogen peroxide.

6. A method for disinfecting at least one surface, comprising at least the following steps of:
a) arranging a device (10) according to any of claims 1 to 5,
b) generating a water vapor flow,
c) preheating and microatomizing said disinfecting composition,
d) injecting into the water vapor flow said disinfecting composition comprising at least one disinfectant, in preheated and microatomized form, in order to obtain a mixture in the form of a jet (16) of water vapor and disinfecting composition,
e) projecting said mixture in the form of a jet of water vapor and disinfecting composition at a predetermined distance from said surface by means of said flexible tube.

7. The method according to the preceding claim, **characterized in that**, while the water vapor generator of said device (10) comprises a boiler (3) and a solenoid (4) for controlling the water vapor flow leaving said boiler (3), in step b), the pressure of the water vapor is raised to at least 2.5 10⁵ Pa inside said boiler (3) in continuous use.

8. The method according to claim 6 or 7, **characterized in that** in step b), the water vapor flow is raised to a flow rate of at least 30 g/minute, e.g. at least 40 g/minute, or at least 50 g/minute.

9. The method according to any of claims 7 or 8, **characterized in that** the water vapor flow has a temperature of at least 130 °C inside said boiler (3) in continuous use.

10. The method according to any of claims 6 to 9, **characterized in that** the flow rate of the mixture in the form of the jet (16) of water vapor and disinfecting composition is at least 60 g/minute, preferably at least 80 g/minute, e.g. at least 90 g/minute.

11. The method according to any of claims 6 to 9, **characterized in that** the temperature of the mixture in the form of the jet (16) of water vapor and disinfecting composition is at least 100 °C, preferably at least 110 °C, e.g. at least 120 °C, at the injector of said device (10).

12. The method according to any of claims 6 to 11, **characterized in that**, according to step d), said mixture in the form of the jet (16) is projected for a duration from 1 to 5 seconds.

13. The method according to any of claims 6 to 12, **characterized in that** according to step d), the mixture in the form of the jet (16) is projected onto the surface to be treated at a distance from 10 cm to 100 cm, e.g. from 10 cm to 50 cm.

14. The method according to any of claims 6 to 13, **characterized in that**, in step d), the flow of the disinfecting composition is raised to a flow rate of at least 30 g/minute, e.g. at least 60 g/minute.

15. The method according to any of claims 6 to 14, **characterized in that** the disinfecting composition is an aqueous solution of hydrogen peroxide, said hydrogen peroxide being present in the aqueous solution with a content of 10 % in weight, with respect to the weight of said aqueous solution, in step b) the water vapor flow is raised to a flow rate of 40 g/minute, and in step d) the flow of the disinfecting composition is raised to a flow rate of 60 g/minute.

## Patentansprüche

1. Tragbare Vorrichtung (10) zum Desinfizieren einer Oberfläche, umfassend:
- mindestens einen Wasserbehälter (1; 12),
- mindestens einen Wasserdampfgenerator (3; 11) zum Erzeugen eines Wasserdampfflusses,
- mindestens einen Behälter (5; 13) für eine desinfizierende Zusammensetzung, die mindestens ein Desinfektionsmittel umfasst,
- mindestens eine Düse (7) zum Einspritzen der desinfizierenden Zusammensetzung in den Wasserdampffluss, um eine Mischung in Form eines Strahls (16) aus Wasserdampf und desinfizierender Zusammensetzung auszubilden, wobei die Einspritzdüse ferner eine Kammer zum Vorwärmen und Mikrozerstäuben der desinfizierenden Zusammensetzung umfasst, wobei die Einspritzdüse in den Wasserdampfgenerator integriert ist,
**dadurch gekennzeichnet, dass** sie ferner mindestens einen Schlauch umfasst, der an den Wassergenerator angeschlossen ist und in der Lage ist, die Mischung in Form eines Strahls von Wasser und der desinfizierenden Zusammensetzung in einem vorherbestimmten Abstand von der Oberfläche abzugeben.

2. Vorrichtung (10) nach Anspruch 1, **dadurch gekennzeichnet, dass** der Schlauch (14) mit einem Betätigungsgriff (15) versehen ist, der das Öffnen und/oder Schließen einerseits des Wasserdampfstrahls und andererseits des Einspritzens der desinfizierenden Zusammensetzung in den Wasserdampfstrahl steuert.

3. Vorrichtung (10) nach einem der Ansprüche 1 oder 2, **dadurch gekennzeichnet, dass** das Desinfektionsmittel aus Wasserstoffperoxid, einer Mischung aus Essigsäure und Peressigsäure, Milchsäure, ätherischen Ölen und deren Mischungen ausgewählt wird und bevorzugt Wasserstoffperoxid ist.

4. Vorrichtung (10) nach einem der vorhergehenden Ansprüche, **dadurch gekennzeichnet, dass** das Desinfektionsmittel in der desinfizierenden Zusammensetzung mit einem Gehalt vorliegt, der von 3 bis 15 Gewichtsprozent, bevorzugt von 3 bis 10 Gewichtsprozent, weiter bevorzugt von 5 bis 10 Gewichtsprozent, weiter bevorzugt von 8 bis 10 Gewichtsprozent, im Verhältnis zum Gewicht der Zusammensetzung reicht.

5. Vorrichtung (10) nach einem der vorhergehenden Ansprüche, **dadurch gekennzeichnet, dass** die desinfizierende Zusammensetzung eine wässrige Lösung von Wasserstoffperoxid ist.

6. Verfahren zum Desinfizieren mindestens einer Oberfläche, umfassend mindestens die folgenden Schritte:
a) Anordnen einer Vorrichtung (10) nach einem der Ansprüche 1 bis 5,
b) Erzeugen eines Wasserdampfflusses,
c) Vorwärmen und Mikrozerstäuben der desinfizierenden Zusammensetzung,
d) Einspritzen in den Wasserdampffluss der desinfizierenden Zusammensetzung, die mindestens ein Desinfektionsmittel umfasst, in vorgewärmter und mikrozerstäubter Form, um eine Mischung in Form eines Strahls (16) von Wasserdampf und desinfizierender Zusammensetzung zu erzielen,
e) Spritzen der Mischung in Form eines Strahls von Wasserdampf und desinfizierender Zusammensetzung in einem vorherbestimmten Abstand von der Oberfläche anhand des Schlauches.

7. Verfahren nach dem vorhergehenden Anspruch, **dadurch gekennzeichnet, dass**, während der Wasserdampfgenerator der Vorrichtung (10) einen Kessel (3) und ein Magnetventil (4) umfasst, um den Wasserdampffluss zu steuern, der den Kessel (3) verlässt, in Schritt b) der Druck des Wasserdampfes in dem Kessel (3) bei Dauergebrauch auf mindestens 2,5 10⁵ Pa angehoben wird.

8. Verfahren nach Anspruch 6 oder 7, **dadurch gekennzeichnet, dass** in Schritt b) der Wasserdampffluss auf einen Durchsatz von mindestens 30 g/Minute, beispielsweise mindestens 40 g/Minute oder mindestens 50 g/Minute, angehoben wird.

9. Verfahren nach einem der Ansprüche 7 oder 8, **dadurch gekennzeichnet, dass** der Wasserdampffluss in dem Kessel (3) bei Dauergebrauch eine Temperatur von mindestens 130 °C aufweist.

10. Verfahren nach einem der Ansprüche 6 bis 9, **dadurch gekennzeichnet, dass** der Durchsatz der Mischung in Form des Strahls (16) aus Wasserdampf und desinfizierender Zusammensetzung mindestens 60 g/Minute, bevorzugt mindestens 80 g/Minute, beispielsweise mindestens 90 g/Minute, beträgt.

11. Verfahren nach einem der Ansprüche 6 bis 9, **dadurch gekennzeichnet, dass** die Temperatur der Mischung in Form des Strahls (16) aus Wasserdampf und desinfizierender Zusammensetzung mindestens 100 °C, bevorzugt mindestens 110 °C, beispielsweise mindestens 120 °C, an der Einspritzdüse der Vorrichtung (10) beträgt.

12. Verfahren nach einem der Ansprüche 6 bis 11, **dadurch gekennzeichnet, dass** gemäß Schritt d) die Mischung in Form des Strahls (16) 1 bis 5 Sekunden lang gespritzt wird.

13. Verfahren nach einem der Ansprüche 6 bis 12, **dadurch gekennzeichnet, dass** gemäß Schritt d) die Mischung in Form des Strahls (16) in einem Abstand von 10 cm bis 100 cm, beispielsweise von 10 cm bis 50 cm, auf die zu behandelnde Oberfläche gespritzt wird.

14. Verfahren nach einem der Ansprüche 6 bis 13, **dadurch gekennzeichnet, dass** in Schritt d) der Fluss der desinfizierenden Zusammensetzung auf einen Durchsatz von mindestens 30 g/Minute, beispielsweise mindestens 60 g/Minute, gebracht wird.

15. Verfahren nach einem der Ansprüche 6 bis 14, **dadurch gekennzeichnet, dass** die desinfizierende Zusammensetzung eine wässrige Lösung aus Wasserstoffperoxid ist, wobei das Wasserstoffperoxid in der wässrigen Lösung mit einem Gehalt von 10 Gewichtsprozent im Verhältnis zum Gewicht der wässrigen Lösung vorliegt, in Schritt b) der Wasserdampffluss auf einen Durchsatz von 40 g/Minute gebracht wird, und in Schritt d) der Fluss der desinfizierenden Zusammensetzung auf einen Durchsatz von 60 g/Minute gebracht wird.
